Europäisches Patentamt

**European Patent Office**

**Office européen des brevets**

(19)

(11) Numéro de publication: **0 282 363 B1**

# FASCICULE DE BREVET EUROPEEN

(12)

(45) Date de publication de fascicule du brevet: **09.09.92**   (51) Int. Cl.5: **A61K 37/64**, C07K 3/12

(21) Numéro de dépôt: **88400235.3**

(22) Date de dépôt: **02.02.88**

(54) **Procédé d'obtention d'un concentré d'alpha 1-antitrypsine à partir d'une fraction de plasma humain et son utilisation à titre de médicament.**

(30) Priorité: **05.02.87 FR 8701403**

(43) Date de publication de la demande:
**14.09.88 Bulletin 88/37**

(45) Mention de la délivrance du brevet:
**09.09.92 Bulletin 92/37**

(84) Etats contractants désignés:
**AT BE CH DE ES FR GB GR IT LI LU NL SE**

(56) Documents cités:
**EP-A- 0 035 204**
**US-A- 4 496 689**

**BIOLOGICAL ABSTRACTS, vol. 62, 15 octobre 1976, résumé no. 44859, Philadelphia, PA., US; C.B. GLASER et al.: "Isolation and characterization of alpha-1-antitrypsin from the cohn fraction IV-I of human plasma" & PREP BIOCHEM 5(4): 333-348 1975**

(73) Titulaire: **CENTRE REGIONAL DE TRANSFU-SION SANGUINE DE LILLE**
**19-21 rue Camille Guérin**
**F-59012 Lille(FR)**

(72) Inventeur: **Burnouf Thierry**
**5, rue du Dr. Schaffner**
**F-59136 Wavrin(FR)**

(74) Mandataire: **Lepeudry-Gautherat, Thérèse et al**
**CABINET LEPEUDRY, 52, avenue Daumesnil**
**F-75012 Paris(FR)**

BIOLOGICAL ABSTRACTS, vol. 71, no. 11, 1981, résumé no. 71219, Philadelphia, PA., US; J.P. SALIER et al.: "Purification of the human serum inter-alpha-trypsin inhibitior by zinc chelate and hydrophobic interaction chromatographies" & ANAL BIOCHEM 109(2): 273-283 1980(recd. 1981)

BIOLOGICAL ABSTRACTS, vol. 65, no. 12, 15 juin 1978, résumé no. 70099, Philadelphia, PA., US; P. MUSIANI et al.: Human serum alpha-I-antitrypsin (aiAT); Isolation and characterization" & ACTA MED ROMANA 14(3): 202-216 1976(recd. 1977)

JOURNAL OF CHROMATOGRAPHY, vol. 296, pages 221-229, Elsevier Science Publishers B.V., Amsterdam, NL; G. GUNZER et al.: "Purification of alpha-1-proteinase inhibitor by triazine dye affinity chromatography, ion-exchange chromatography and gel filtration on fractogel TSK"

BIOCHEMISTRY, vol. 15, no. 4, février 1976, pages 798-804; P. MUSIANI et al.: "Isolation, chemical, and physical properties of alpha-I-antitrypsin"

CHEMICAL ABSTRACTS, 1963, colonne 4806b, Columbus, Ohio, US; P. KISTLER et al.: "Large-scale production of human plasma protein; eight year experience with the ethanol fractionation procedure of Nitschmann, Kistler, and Lerbier" & VOX SANGUINIS 7, 414-24(1962)

Cohn, E.J. et al.: A system in the separation of the components of human plasma. J.AM. Chem. Soc. 72: 465,1950

## Description

La présente invention concerne un procédé d'obtention d'un concentré d'α 1-antitrypsine à partir d'une fraction de plasma humain, le produit ainsi obtenu et son utilisation à des fins thérapeutiques.

Les inhibiteurs de protéase du plasma jouent un rôle primordial dans le contrôle de l'action des enzymes protéolytiques et possèdent ainsi une action régulatrice dans la formation des tissus conjunctifs, ainsi que dans la coagulation, la fibrinolyse et la formation de kinine. L'α 1-antitrypsine ou AAT, glycoprotéine produite dans le foie, est ainsi un inhibiteur d'élastase neutrophile, enzyme mise en jeu dans la protéolyse des tissue conjonctifs, plus particulièrement du poumon. Une déficience en AAT peut conduire à des affections pulmonaires telles que l'emphysème.

On a donc pensé à soigner des patients présentant une telle déficience par administration d'AAT. Toutefois il est nécessaire de disposer à cette fin d'un produit de haute pureté susceptible d'être obtenu avec un rendement significatif dans les centres de traitement du plasma humain.

On a ainsi proposé des techniques de préparation d'AAT, qui n'ont toutefois permis d'aboutir qu'à des degrés de pureté assez faibles, de l'ordre de 60 à 70 % et qui nécessitant l'utilisation de fractions de plasma peu courantes. De telles techniques sont notamment décrites dans l'article de Coan et al ( Vox Sang. 48 33-342, et dans celui de Glaser et al (Anal. Biochem. 124, 364-371, 1982).

Il serait donc intéressant de disposer d'une technique de préparation d'AAT de haute pureté, susceptible d'être mise en oeuvre à l'échelle industrielle utilisant des fractions de plasma aisément disponibles dans les centres de fractionnement importants.

La Demanderesse a ainsi découvert qu'il était possible de préparer de l'AAT de degré de pureté supérieur ou égal à 80% par des techniques chromatographiques utilisant une fraction de plasma couramment obtenue dans les centres de fractionnement produisant de l'albumine et qui est en fait un sous-produit, précédemment rejeté, de la préparation de celle-ci.

L'invention concerne donc un procédé de préparation d'un concentré d' α 1-antitrypsine (AAT) à partir d'une fraction de plasma humain, caractérisé en ce qu'on utilise comme fraction de plasma un surnageant A ou A + I selon la nomenclature de Kistler et Nitschmann : (Vox Sang; 7, 414-424 ; c'est-à-dire le surnageant obtenu après précipitation à l'éthanol soit en une seule étape soit en deux étapes successives) et que l'on soumet à une purification par voie chromatographique jusqu'à obtention d'un concentré ayant une teneur en AAT de 80% au moins.

Ce procédé permet d'obtenir, avec un rendement de l'ordre de 50%, une AAT pure à 80% au moins, et ayant conservé une forte action inhibitrice à l'encontre de la trypsine et de l'élastase.

La préparation du surnageant utilisé se fait par précipitation à l'éthanol, à un pH voisin de la neutralité ou légèrement acide, d'un plasma ayant été soumis à un traitement de cryoprécipitation.

On met avantageusement en oeuvre une double précipitation à l'éthanol pour séparer un surnageant de caractéristiques correspondant au surnageant A selon la nomenclature de Kistler et Nitschmann que l'on sépare de l'éthanol, par exemple par diafiltration.

On peut également utiliser un surnageant obtenu par une précipitation simple à l'éthanol, correspondant au surnageant A + I selon la nomenclature de Kistler et Nitschmann rappelée ci-dessus.

A partir du surnageant obtenu on sépare par chromatographie d'échange d'anions une fraction riche en albumine, qui peut être utilisée pour récupérer cette protéine, et une fraction riche en AAT. On purifie ensuite celle-ci après concentration et dialyse et éventuellement addition d'un stabilisant, par chromatographie d'exclusion stérique pour éliminer les contaminants de poids moléculaire élevé.

Après cette purification on inactive de préférence les virus éventuellement présents, tels que les virus de l'hépatite ou du SIDA. On peut réaliser cette inactivation par tout moyen connu de l'homme de l'art et en particulier par chauffage à l'état liquide, en présence éventuellement d'un stabilisant approprié, permettant de protéger l'AAT contre la formation d'aggregats et la dénaturation. On peut également réaliser cette inactivation par chauffage à l'état lyophilisé, traitement chimique (par exemple traitement au solvant-détergent) ou irradiation (par exemple irradiation UV).

Après diafiltration pour éliminer le stabilisant éventuellement présente et ajuster la teneur en protéine à environ 20 g/l on met le concentré en ampoules et on lyophilise. Cette concentration peut éventuellement être portée à 60 g/l afin d'assurer une dose de 1 g par flacon de 20 ml.

Le produit ainsi obtenu peut être utilisé pour le traitement des affections pulmonaires, notamment chez les patients présentant une défience marquée en AAT.

La posologie à utiliser est d'environ 60 mg/kg de poids corporel par semaine, par voie intraveineuse. Il est également possible d'administrer ce produit sous forme d'aérosol.

L'exemple suivant illustre l'invention sans toutefois en limiter la portée.

A) Préparation du concentré.

On prépare le surnageant riche en albumine et AAT en précipitant à l'éthanol à 10 %, à pH 7,4, un plasma ayant subi une cryoprécipitation et en reprécipitant le premier surnageant obtenu par de l'éthanol à 19 %, à pH 5,85, à une température de 5 ± 1°C.

Après centrifugation, on élimine l'éthanol par diafiltration et on dilue jusqu'à une teneur en protéines d'environ 15 g/l. On sépare l'albumine de l'AAT sur une colonne de DEAE - SepharoseCL-6B Ⓜ Fast Flow (Pharmacia) équilibrée à pH 5,2 à 6 par un tampon à l'acétate de sodium. Par élution à l'acétate de sodium 0,15 M on récupère une fraction riche en AAT. La fraction d'AAT est stabilisée dès sa sortie de colonne DEAE par une remontée à pH 6,5 et par adjonction de glycocolle à raison de 5 g/l. Après concentration à 45 g/l environ et dialyse, on purifie par chromatographie sur Séphacryl S-200 Ⓜ .

On ajoute de préférence du glycocolle à raison de 1 g/l (concentration finale) afin de stabiliser l'AAT lors de la lyophilisation et pour favoriser la mise en solution du produit lors de l'utilisation.

Après diafiltration pour ajuster la teneur en protéines à environ 25 g/l on met le concentré en ampoules de 50 ml et on lyophilise.

B) Analyse du concentré.

On détermine par immunophélométrie (sur ICS Beckmann) la composition en protéines du produit, qui est la suivante (exprimé en g/l) :

| Protéines | 28 |
|---|---|
| AAT | 24,8 |
| Albumine | 0,65 |
| Haptoglobine | 0,6 |
| $\alpha_2$ HS glycoprotéine | 1,3 |
| AT III | 0,18 |
| $I_G$A | 0,08 |
| Transferrine | 0,1 |
| $\alpha_2$ macroglobuline | 0 |

Les contaminants les plus importants sont l'$\alpha_2$ HS glycoprotéine (environ 4,6 %), l'albumine (2,3 %), l'haptoglobine (2,1%) et l'antithrombine III (0,6 %).

Ces résultats sont confirmés par contrôles électrophorétiques.

C) Détermination de l'activité du concentré

1) Capacité inhibitrice de la trypsine

Cette caractéristique a été déterminée selon deux méthodes, la méthode "BAPNA" et la méthode "Chromozyme" décrites respectivement par Erlanger et al. (Arch. Biochem. Biophys. 95, 271-278, 1961) et Mullins et al. (Clin. Chem.30, 1857-1860, 1984).

Les résultats obtenus selon les deux méthodes après correction pour tenir compte de la concentration en AAT, correspondant aux valeurs connues pour l'AAT sérique native soit 1,3 ± 0,1.

2) Capacité inhibitrice de l'élastase

Cette caractéristique a été déterminée selon la méthode décrite par Beatty et al (J. Biol. Chem. 255, 3931-3934, 1980).

La valeur obtenue pour la constante d'association (kass) de l'AAT produite avec de l'élastase porcine est de 2,24 $10^5$ $M^{-1}$ $s^{-1}$. Cette valeur est semblable à celle de l'AAT sérique native, telle qu'indiquée notamment dans la publication de Beatty et al référencée ci-dessus.

Les essais pharmacologiques et cliniques réalisés ont montré que l'AAT produite selon l'invention était dépourvue de toxicité et parfaitement tolérée par voie intraveineuse. Sa durée de demi-vie est de l'ordre de 6 à 7 jours, ce qui est largement supérieur aux résultats obtenus pour des concentrés préparés par d'autres procédés et notamment à partir de fractions de plasma différentes.

4

EP 0 282 363 B1

**Revendications**

1. Procédé de préparation d'un concentré d'α 1-antitrypsine (AAT) à partir d'une fraction de plasma humain, caractérisé en ce qu'on utilise comme fraction de plasma un surnageant A ou A + I selon la nomenclature de Kistler et Nitschmann (c'est-à-dire obtenu après précipitation à l'éthanol, soit en deux étapes soit en une seule étape) et que l'on soumet ce surnageant à une purification par voie chromatographique jusqu'à obtention d'un concentré ayant une teneur en AAT de 80 % au moins.

2. Procédé selon la revendication 1, caractérisé en ce que la purification est faite par deux séparations chromatographiques successives.

3. Procédé selon la revendication 2, caractérisé en ce qu'on sépare par chromatographie d'échange d'anions le surnageant de la précipitation à l'éthanol en une fraction riche en albumine et une fraction riche en AAT et on purifie la fraction riche en AAT par chromatographie d'exclusion stérique.

4. Procédé selon la revendication 3, caractérisé en ce qu'on ajoute un stabilisant à la fraction riche en AAT avant sa purification par chromatographie d'exclusion stérique.

5. Procédé selon la revendication 4, caractérisé en ce que le stabilisant est le glycocolle.

6. Procédé selon la revendication 5, caractérisé en ce que le glycocolle est ajouté à raison de 5 g/l.

**Claims**

1. Method of preparing an α 1-antitrypsin (AAT) concentrate from a human plasma fraction, characterised in that there is used as the plasma fraction a supernatant A or A + I according to the nomenclature of Kistler and Nitschmann (that is to say obtained after precipitation with ethanol, either in two steps or in a single step) and that the supernatant is subjected to purification by chromatography until a concentrate having an AAT content of at least 80% is obtained.

2. Method according to claim 1, characterised in that the purification is achieved by two successive chromatographic separations.

3. Method according to claim 2, characterised in that the supernatant from the precipitation with ethanol is separated by anion exchange chromatography into an albumin-rich fraction and an AAT-rich fraction, and the AAT-rich fraction is purified by size exclusion chromatography.

4. Method according to claim 3, characterised in that a stabiliser is added to the AAT-rich fraction before it is purified by size exclusion chromatography.

5. Method according to claim 4, characterised in that the stabiliser is glycocoll.

6. Method according to claim 5, characterised in that glycocoll is added at the rate of 5 g/l.

**Patentansprüche**

1. Verfahren zum Herstellen eines Konzentrats von Alpha-1-Antitrypsin (AAT) von menschlicher Plasmafraktion, **dadurch gekennzeichnet,** daß man als Plasmafraktion das Aufschwimmende A oder A + I entsprechend der Nomenklatur von Kistler und Nitschmann verwendet (d.h., das man mit Abscheidung in Äthanol erhält, sei es in zwei Schritten oder in nur einem Schritt), und daß man dieses Aufschwimmende einer Reinigung auf chromatografischem Weg bis zur Erzielung einer Konzentration eines AAT-Gehalts von wenigstens 80% unterzieht.

2. Verfahren nach Anspruch 1, **dadurch gekennzeichnet,** daß die Reinigung durch zwei aufeinanderfolgende chromatografische Trennungen ausgeführt wird.

3. Verfahren nach Anspruch 1, **dadurch gekennzeichnet,** daß man durch Anionenaustausch-Chromatografie das Aufschwimmende der Abscheidung in Äthanol in einer an Albumin reichen Fraktion und einer an AAT reichen Fraktion abtrennt und daß man die an AAT reiche Fraktion durch sterische Ausschlußchromatografie reinigt.

4. Verfahren nach Anspruch 3, **dadurch gekennzeichnet,** daß man der an AAT reichen Fraktion vor seiner Reinigung durch sterische Ausschlußchromatografie einen Stabilisator hinzufügt.

5. Verfahren nach Anspruch 4, **dadurch gekennzeichnet,** daß der Stabilisator Glycin ist.

6. Verfahren nach Anspruch 5, **dadurch gekennzeichnet,** daß Glycin in einer Menge von 5 g/l hinzugesetzt wird.